# EUROPÄISCHE PATENTSCHRIFT

(11) **EP 0 663 811 B1**
(45) Veröffentlichungstag und Bekanntmachung des Hinweises auf die Patenterteilung: **12.07.2000**
(21) Anmeldenummer: 93922938.1
(22) Anmeldetag: 12.10.1993
(51) Int. Cl.: A61F 2/36

(54) **STIEL EINER FEMURKOMPONENTE EINER HÜFTGELENKSENDOPROTHESE**
PIN OF A FEMUR COMPONENT OF A HIP-JOINT ENDOPROSTHESIS
TIGE D'UN CONSTITUANT FEMORAL D'UNE ENDOPROTHESE DE L'ARTICULATION DE LA HANCHE

(30) Priorität: 12.10.1992 DE 4234351
(43) Veröffentlichungstag der Anmeldung: 26.07.1995
(73) Patentinhaber: DRAENERT, Klaus, Dr.med., D-81545 München (DE)
(72) Erfinder: DRAENERT, Klaus, Dr.med., D-81545 München (DE)
(86) Internationale Anmeldenummer: EP9302804
(87) Internationale Veröffentlichungsnummer: WO9408534

(56) Entgegenhaltungen:
- EP-A- 0 222 236
- EP-A- 0 240 815
- EP-A- 0 477 113
- WO-A-91/18560
- DE-A- 3 829 361
- DE-C- 3 913 874
- FR-A- 2 583 286
- FR-A- 2 618 667
- US-A- 4 435 854

## Beschreibung

Die Erfindung betrifft einen Stiel oder Schaft einer Femurkomponente einer Hüftgelenksprothese, die zementfrei oder mit Knochenzement eingesetzt werden kann, sowie eine einen derartigen Stiel aufweisende Femurkomponente.

In der Orthopädie und Chirurgie des Bewegungsapparates sind die Gelenkersatzoperationen heute nicht mehr wegzudenken. Gleichwohl sind die Ergebnisse dieser Operationen sehr unterschiedlich, sowohl im Hinblick darauf, ob die Prothesenkomponente zementfrei oder mit Zement eingesetzt wird, als auch im Hinblick auf die einzelnen Kliniken.

Die einzelnen Ergebnisse sind oft wenig vergleichbar und auch an ein und derselben Klinik sehr oft wenig reproduzierbar. Dies hat schließlich dazu geführt, daß eine ganze Reihe von Prothesenkomponenten entwickelt wurden, was am Beispiel des Hüftgelenkes und hierbei am Beispiel der Femurkomponente solche Ausmaße angenommen hat, daß es heute bereits über 400 verschiedene Versionen derartiger Komponenten auf dem Markt gibt.

Eine der Hauptgründe für diese große Zahl an verschiedenen Prothesendesigns ist die große Varianz in der Morphologie individueller Knochen und auch die große Varianz in den Eigenschaften dieser einzelnen Knochen. Die Varianz der normalen Anatomie und Physiologie wird noch erweitert durch das breite Spektrum der pathologischen Veränderungen an den Knochen, die letztlich auch zum Verschleiß des Gelenkes geführt haben, soweit nicht ein Unfall die Ursache der Zerstörung des Gelenkes ist.

Aufgrund vieler ungelöster Probleme in Hinsicht auf die Anpassung an die Natur des Knochens gibt es heute Krankheitsbilder, die als "Prothesenkrankheit" zusammengefaßt sind. Eine der Hauptursachen hierfür liegt jedoch darin, daß es bislang nicht gelungen ist, eine Standardprothese zu entwickeln, die sich optimal einer möglichst großen Zahl von physiologischen Formen des proximalen Femurendes anpaßt, ohne daß hier sehr viel Knochen entfernt werden muß, wenn das knöcherne Bett der Prothese angepaßt wird.

So gibt es verschiedene Versuche, wie beispielsweise in der EP-A-0 038 908 und in der US-A-4 435 854, in denen dargelegt wird, wie man sich der Markhöhle des Knochens anatomisch adaptieren und physiologische Krümmungen anstreben kann.

Versuche mit natürlichen Femora haben jedoch gezeigt, daß diese physiologischen Krümmungen nicht auf dem physiologischen Wege in die Markhöhle eingebracht werden können, ohne daß es zunächst zu einer Behinderung bei der Einführung der Femurkomponente kommt, d. h. es gelingt nicht, die anatomisch adaptierte Form ohne entsprechende Aufbereitung des Zugangsweges oder Implantationsweges in den Knochen einzuführen.

In der WO-A-9118560 wird der Versuch unternommen, durch Verwringung des Stieles sich der Anatomie des Femurs anzupassen. Dies gelingt jedoch nur teilweise, und zwar in Bezug auf die sogenannte Antetorsion des Schenkelhalses, und auch da nur unvollständig.

Der Erfindung liegt somit die Aufgabe zugrunde, einen Femurstiel einer Hüftgelenksprothese bereitzustellen, der optimal an die Markhöhle des Knochens angepaßt, insbesondere im implantierten Zustand optimal zentriert ist, und der leicht in die Markhöhle des Knochens einführbar ist.

Bei den im Rahmen der Erfindung durchgeführten systematischen Untersuchungen zur Lösung des genannten Problems wurde gefunden, daß bisher ein wesentliches Kriterium bei der Entwicklung der Prothesenschäfte übersehen worden war. Bei den Untersuchungen wurde eine Torquierung bzw. Verwringung des Knochens aufgrund der außen am Knochen ansetzenden, sehr starken Muskelkräfte festgestellt. Diese wissenschaftliche Erkenntnis liegt der Erfindung zugrunde, durch die das Problem der Einpassung der Femurkomponente in den Knochen auf überraschende Weise sehr einfach und preiswert zu lösen ist.

Zur Lösung der vorstehenden Aufgabe geht die Erfindung zunächst von dem Grundgedanken aus, daß der Markkanal sich in derselben Weise verwringt wie das zylindrische Rohr des Knochens und somit auch die sich im Innern des Markkanales befindlichen Knochenstrukturen, nämlich die Fachwerke der Spongiosabälkchen. So wurde erfindungsgemäß gefunden, daß das Markrohr des proximalen Femur eindeutig durch eine Achse definiert werden kann, und daß diese Achse auch die ideale Konstruktionsachse für die Prothese selbst und für den Zugangsweg der Prothese, d.h. den Operationsweg, darstellt. Es wurde außerdem gefunden, daß der Schenkelhals dreidimensional schräg in das Femurmarkrohr einmündet und daß seine Strukturen sich im Innern des Knochens im Markrohr des Femurschaftzylinders wiederfinden und dadurch Kompartimente im Querschnitt des proximalen Femurknochens bestimmen.

Die Querschnitte sowohl des Markkanales als auch des Schaftzylinders entsprechen im proximalen Teil, d.h. in der Höhe der Einmündung des Schenkelhalses, einem Queroval bzw. einer Ellipse mit der längeren Achse in medial-lateraler Richtung. Im Bereich der Muskelursprünge am kleinen Trochanter gehen die Querschnitte dann in eine mehr rechteckige Konfiguration über, welche im gesamten proximalen Bereich der Metaphyse beibehalten wird, bis zum Übergang der Diaphyse, als welche das eigentliche zylindrische Schaftrohr des Femur bezeichnet wird. Es wurde gefunden, daß dieses im Beginn, d.h. proximal, im Querschnitt eher einem Queroval gleicht und sich dann langsam über die Abschnitte der proximalen Femurhälfte bis zu dem Bereich, in dem im implantierten Zustand die Prothesenspitze angeordnet ist, in ein Sagittaloval bzw. eine Ellipse mit der längeren Achse in ventral-dorsaler Richtung einstellt. Aus der dreidimensionalen Rekonstruktion dieser Abschnitte konnte eine Verwringung des Markkanales ermittelt werden. Erfindungsgemäß wird die Form des Femurschafts optimal an diese Form des Markkanales angepaßt. Der Querschnitt des erfindungsgemäßen Femurstiels ist in einer Ausführungsform im proximalen Bereich quer-oval und "dreht" sich nach distal derart, daß er im distalen Bereich sagittal-oval ist.

Die Morphologie des Femur, welche in der Arbeit von Noble, "The anatomic basis of femoral component design", Clin. Orthop. Rel. Res. 235 (1988), 148-165, erarbeitet worden war, ließ erkennen, daß im axialen Strahlengang im Röntgenbild das Femur eine S-förmige Schwingung aufweist, derart, daß proximal der Krümmungsmittelpunkt des Schwingungsradius vor dem Femur, d.h. ventral, und distal der Krümmungsmittelpunkt dorsal des Femur zu liegen kommt. Um eine Prothese optimal im Femurmarkkanal zu konzentrieren, wäre demnach eine Anpassung an diese S-förmige Schwingung wünschenswert.

Die exakte Analyse im Rahmen der vorliegenden Erfindung ließ jedoch überraschenderweise erkennen, daß vor allem in demjenigen Bereich, in dem hauptsächlich Kraft zwischen dem Knochen und der Prothese übertragen wird, d.h. auf der Dorsalseite des Femurmarkkanals sowie medial und antero-medial, die dorsale Wand geradlinig verläuft und in der Weise definiert werden kann, daß die Rückwand des Schenkelhalses, welche als Isthmus-colli auf dem seitlichen Röntgenbild des Femur augenscheinlich wird, und die Rückwand des Femurmarkkanales eine Ebene bilden, zu der parallel die Implantationsachse definiert werden muß. Auf diese Weise ist ein geradliniger Zugang zur Markhöhle definiert, der auch im Design des erfindungsgemäßen Prothesenstieles seinen Niederschlag findet.

Die Erfindung beruht auf der Erkenntnis, daß ein der Markhöhle angepaßter Körper, beispielsweise der Femurstiel einer Hüftgelenksendoprothese, in sich in derselben Weise wie der Markkanal bzw. der Knochen verwrungen bzw. torquiert sein muß, damit er ohne anzustoßen entlang der Implantationsachse eingeführt werden kann. Diese Verwringung oder Verdrehung erfolgt bei einem linken Stiel vorzugsweise von distal nach proximal im Gegenuhrzeigersinn, bei einem rechten Stiel im Uhrzeigersinn. Es hat sich gezeigt, daß ein zunächst S-förmig geschwungener Schaft, nachdem er gerade gerichtet ist, weiter in den Femurmarkkanal einsinkt als der ursprünglich S-förmig adaptierte Schaft, der ansonsten ähnlich ausgeführt ist. Ein geradlinig ausgeführter, aber in sich verwrungener Schaft kann, im Gegensatz zum ansonsten identischen S-förmigen Schaft, vollständig in den Markkanal eingeführt werden, ohne daß weiterer Knochen entfernt werden muß. Für das Design der erfindungsgemäßen Prothese wird hieraus abgeleitet, daß diese auf der Dorsalseite vorzugsweise geradlinig konstruiert werden muß und/oder daß der Prothesenkörper in sich von proximal nach distal verwrungen werden muß, und zwar um etwa 15 bis 195°, vorzugsweise um etwa 45 bis 135°, besonders bevorzugt um etwa 90°, bezogen auf den proximalen Abschnitt des Femur, in den die Femurkomponente implantiert wird. Die Torsion erstreckt sich also beispielsweise über die proximalen 21 cm des Femur. Auch geringere Verwringungen erleichtern schon ganz wesentlich das Einführen der Prothese. Darüber hinaus wird auf diese Weise erreicht, daß sich das Kopfzentrum im Sinne einer Anteversion des Femur anatomisch einstellt.

Der erfindungsgemäße Femurstiel weist vorzugsweise über seine gesamte Länge, in jedem Fall in seinem proximalen Bereich eine konkave Ausnehmung oder Rinne oder eine Abflachung auf, die sich schrauben- oder wendelförmig in Längsrichtung des Femurstiels erstreckt. Im proximalen Bereich des Schafts befindet sich die Ausnehmung, Rinne oder Abflachung (nachstehend zusammenfassend als "Ausnehmung" bezeichnet) auf der dorsalen Seite des Schafts und dreht sich in Distalrichtung wendelförmig zur lateralen Seite des Schafts. Durch diese Ausnehmung kann ebenfalls eine Verwringung des Femurschafts definiert werden.

Die Erfindung beruht ferner auf der Erkenntnis, daß sich die Vorderwand des Prothesenstiels - im axialen, seitlichen Strahlengang des Röntgenbildes gesehen - dem Verlauf des Schenkelhalses anpassen muß, in dem Sinne, daß die Fläche der Vorderwand gekrümmt zu fertigen ist. Der Krümmungsmittelpunkt liegt dabei vor dem Femur, d.h. ventral, und die Krümmung weist Radien zwischen etwa 60 und 180 mm, vorzugsweise zwischen 80 und 150 mm, besonders bevorzugt 120 bis 130 mm, auf. Besonders vorteilhaft ist ein parabelförmiger Verlauf der Krümmung von distal nach proximal, so daß sich der Krümmungsradius in dieser Projektion nach proximal kontinuierlich verringert.

In Ansicht von ventral bzw. von dorsal, d.h. im anterioposterioren Strahlengang ist der Schaft an seiner medialen Seite gekrümmt, und hierbei liegt der Krümmungsmittelpunkt medial vom Femur, und die Krümmung weist Radien zwischen etwa 15 und 85 mm, vorzugsweise zwischen 25 und 55 mm, auf. Auch hier verläuft die Krümmung vorzugsweise parabelförmig derart, daß der Krümmungsradius von distal nach proximal abnimmt. Vorzugsweise zeigen Krümmungsradien von etwa 36 bis 40 mm optimale Anpassungen an die Kraftübertragungsebene, innerhalb derer das Rotationszentrum beschrieben ist, wie in der PCT/EP 92/01925 dargestellt.

Bei den der Erfindung zugrunde liegenden Untersuchungen konnte vor allem beim Treppensteigen und beim Aufstehen aus der sitzenden Position aufgrund der histologischen Befunde eine sehr starke Retrotorsionskomponente nachgewiesen werden. Die kraftübertragenden Oberflächen wurden eingehend analysiert. Aus dieser Analyse ergibt sich eine Oberflächengestaltung des Prothesenstieles, die sich auf der Dorsalseite von der Gestaltung der Ventralseite unterscheidet. Auf der Dorsalseite ist die Oberfläche nicht nur axial geradlinig, sondern außerdem im Querschnitt konkav gestaltet. Auf der Ventralseite ist die Oberfläche im Querschnitt konvex ausgebildet. Die kraftübertragenden Oberflächen befinden sich vor allem im proximalen Bereich der Prothese. Es konnte gezeigt werden, daß sich durch die genannte Oberflächengestaltung beim Design der Prothese in überraschender Weise bei einer zementierten Komponente eine gleichmäßige Zementschichtdicke um den Prothesenumfang herum ergibt. Bei einer zementfreien Komponente führt die erfindungsgemäße Oberflächengestaltung des Prothesenstiels zu einer sehr viel größeren Kontaktfläche zwischen Implantat und Knochen. Ferner muß beim erfindungsgemäßen Design der Prothesenoberfläche nur sehr wenig Knochen vor der Implantation entfernt werden, beispielsweise mit einer Diamanthohlschleife gemäß DE-A-32 02 193, um den Prothesenstiel verankern zu können. Dies bedeutet für den Patienten, daß nur sehr wenig eigene Knochensubstanz geopfert werden muß und daß diese darüber hinaus, bei Verwendung von Diamanthohlschleifen, auch wieder für Knochentransplantate zur Verfügung steht. Da der Schenkelhalskopf bei einer Schaftimplantation abgesetzt wird, der Rest des Schenkelhalses mit seiner sehr kompakten dorsalen Wand vom Prothesenstiel durchbrochen werden muß, muß bei allen geraden Stielen, die nicht torquiert sind, diese dorsale Wand entfernt werden; dagegen hat sich gezeigt, daß bei der Implantation des erfindungsgemäßen verwrungenen Stiels, der auf seiner dorsalen Seite konkav ist, die dorsale Wand intakt bleibt.

Aus den Querschnitten des proximalen Femur ergibt sich auch eine knaufförmige Auftreibung des proximalen erfindungsgemäßen Prothesenstieles, welche bislang bei den herkömmlichen Prothesen nicht beobachtet werden konnte. In Begriffen der Masse der Prothese gesprochen kann auf diese Weise erreicht werden, daß ein Großteil der Masse proximal und medial konzentriert werden kann und die Prothese nach distal schlanker als herkömmliche Prothesen auslaufen kann. Bei allen Versuchen, derartige erfindungsgemäße Prothesen in den Knochen zu implantieren, ergab sich überraschenderweise praktisch von selbst eine exakte Plazierung des Schaftes in der Mitte des Markkanales, und zwar auch ohne "centralizer". Dadurch, daß bei der erfindungsgemäßen Prothese Implantationsachse und Konstruktionsachse der Prothese zusammenfallen, ermöglicht ein Einschlagloch an der proximalen Austrittsstelle der Konstruktionsachse am Stiel ein reproduzierbares Einsetzen der Prothese.

Der erfindungsgemäße Femurstiel kann auch, insbesondere im proximalen Bereich, derart ausgebildet sein, daß sein Querschnitt U-Form (U-Shape) aufweist, wobei die U-Form nach lateral offen ist und mit ihrer im Medialbereich konvexen Krümmung breit aufliegt. Im weiteren Verlauf nach distal kann der Femurstiel, horizontal gesehen, einen quer-ovalen bis rechteckigen Querschnitt mit deutlich abgerundeten Kanten aufweisen.

Beim erfindungsgemäßen Femurstiel beträgt der Winkel zwischen der Achse des Stiels (Einschlagachse) und der Sahenkelhalsachse, d.h. einer Achse senkrecht zum Querschnitt des Femurstiels an seinem proximalen Ende in der Abbildung im anterio-posterioren Strahlengang, d.h. Ansicht von ventral oder dorsal, in der Regel etwa 126 bis 144°, vorzugsweise etwa 132 bis 140°, besonders bevorzugt etwa 135°. In der Seitenansicht von medial oder lateral (axialer Strahlengang) ist ebenfalls ein Winkel zwischen der Achse des Stiels (Einschlagachse) und der Schenkelhalsachse, die senkrecht auf dem Querschnitt des Femurstiels an seinem proximalen Ende steht, ausgebildet. Dieser Winkel (Antetorsion) beträgt im allgemeinen etwa 0 bis 12°, vorzugsweise etwa 5 bis 10°, besonders bevorzugt etwa 8 bis 9°.

Im einzelnen wird bei der Implantation der erfindungsgemäßen Femurkomponente folgendermaßen vorgegangen: Zunächst wird das Hüftgelenk in der üblichen Weise über einen lateralen Zugang freigelegt und die Kapsel eröffnet und entfernt. Anschließend wird das Femur mit Hilfe eines starken Luxationslöffels in toto luxiert und außenrotiert. In dieser Position kann in Verlängerung der präoperativ geplanten Implantationsachse an der medialen Zirkumferenz des großen Trochanters, leicht dorsal seiner medianen Ebene, die Markhöhle mit einer Diamanthohlschleife eröffnet werden, so daß der Implantationsweg in ganzer Länge freiliegt. Der dabei mit einem Extraktor herausgenommene, aus Kompakta und Spongiosa bestehende Knochenzylinder weist typischerweise eine Länge von nur 8 bis 9 cm auf, wenn bereits die freie Markhöhle erreicht ist. Der Implantationskanal wird daraufhin mit einem zylinderförmigen Führungsinstrument bestückt. Über das Führungsinstrument wird eine Sägeschablone aufgesteckt, die den Weg exakt in der Resektionsebene für die oszillierende Säge freigibt. Damit wird im nächsten Schritt der krankhaft veränderte Hüftkopf abgesetzt, das Führungsinstrument wieder herausgenommen und eine Bohrführung für die Diamanthohlschleife eingesetzt. Entlang dieser kann paßgenau für den ausgewählten Stiel der Prothese mit zylinderförmigen Fräsungen das Spongiosamassiv am Schenkelhals sparsam erweitert werden. Das Calcar femoris wird auf diese Weise erhalten. Als Calcar femoris gilt der Anteil des Schenkelhalses, der in das freie Markrohr des Diaphysenschaftes einstrahlt und als kompakte Struktur im Querschnitt in Höhe des Schenkelhalses noch angetroffen wird, und der den Markraum in ein ventrales und ein dorsales Kompartiment unterteilt. Der Implantationsweg führt durch das ventrale Kompartiment des Markraumes. Es wird nun erneut ein Führungsinstrument in den Eröffnungskanal eingesetzt, und in der präoperativ vorbestimmten Höhe, beispielsweise zwischen 17 und 21 cm distal der Resektionsfläche, eine äußere Bohrführung an das Femur aufgesteckt. Hier wird antero-lateral ein Bohrloch mit 4,5 mm Durchmesser gesetzt und eine Drainagekanüle mit selbstschneidendem Gewinde eingeschraubt, wie in EP-A-305 417 beschrieben. Anschließend wird der Führungsstab herausgenommen, die Markhöhle ausgespült und abgesaugt. Danach wird im Trochantermassiv in Richtung auf die Linea aspera eine zweite Drainagekanüle eingeschraubt und der Knochenzement durch Vakuummischen angemischt. Während des Mischens des Knochenzements durch einen Assistenten wird eine Probeprothese des ausgewählten Types eingesetzt, kontrolliert, anschließend wieder herausgenommen, ein Siegel zur Abdichtung auf das Femur aufgesetzt, die Vakuumpumpe eingeschaltet und der fertig vorkomprimierte Knochenzement mit Hilfe einer Zementpresse oder Zementspritze unter Vakuum in das knöcherne Bett eingesaugt. Dabei wird zunächst distal gesaugt und danach proximal die Saugdrainage geöffnet. Im selben Zuge wird der Prothesenschaft vorsichtig in der Implantationsachse eingeführt und langsam, entsprechend der Viskosität des Knochenzementes, plaziert. Nach Aushärten des Knochenzementes wird auch das Vakuum abgestellt und überstehende Zementreste entfernt. Nach Aufsetzen eines Kugelkopfes wird die Femurkomponente mit dem Oberschenkelknochen reponiert, wobei sowohl im Bereich der Pfanne als auch im Bereich des Femur wieder Vakuum appliziert wird.

Die Erfindung wird nachstehend anhand der Figuren näher erläutert. Es zeigen:
- Figur 1A: eine Ansicht eines erfindungsgemäßen Femurstiels von dorsal,
- Figur 1B: mehrere Querschnitte des Femurstiels von Figur 1A an verschiedenen Positionen in Axialrichtung,
- Figur 1C: eine Ansicht des Femurstiels von Figur 1A von medial,
- Figur 2: eine Ansicht einer Ausführungsform des erfindungsgemäßen Femurstiels (Linksprothese) von dorsal,
- Figur 3: eine Ansicht derselben Ausführungsform von medial,
- Figuren 4A, 4B und 4C: eine Ausführungsform des erfindungsgemäßen Femurstiels (Linksprothese) in CAD-Darstellung von vorne (Strahlengang anterioposterior), von lateral (axialer Strahlengang) und in Draufsicht,
- Figur 5: eine Ausführungsform des erfindungsgemäßen Femurstiels im Schnitt (Strahlengang anterio-posterior, Ansicht von vorne),
- Figur 6: eine Ausführungsform des erfindungsgemäßen Femurstiels mit aufgesetztem Kugelkopf im Schnitt (axialer Strahlengang, Ansicht von lateral),
- Figur 7: eine Ausführungsform eines erfindungsgemäßen Femurstiels mit konischem Hals im Schnitt (axialer Strahlengang, Ansicht von medial), und
- Figur 8: einen Schnitt eines erfindungsgemäßen Femurstiels (Strahlengang anterio-posterior, Ansicht van vorne).

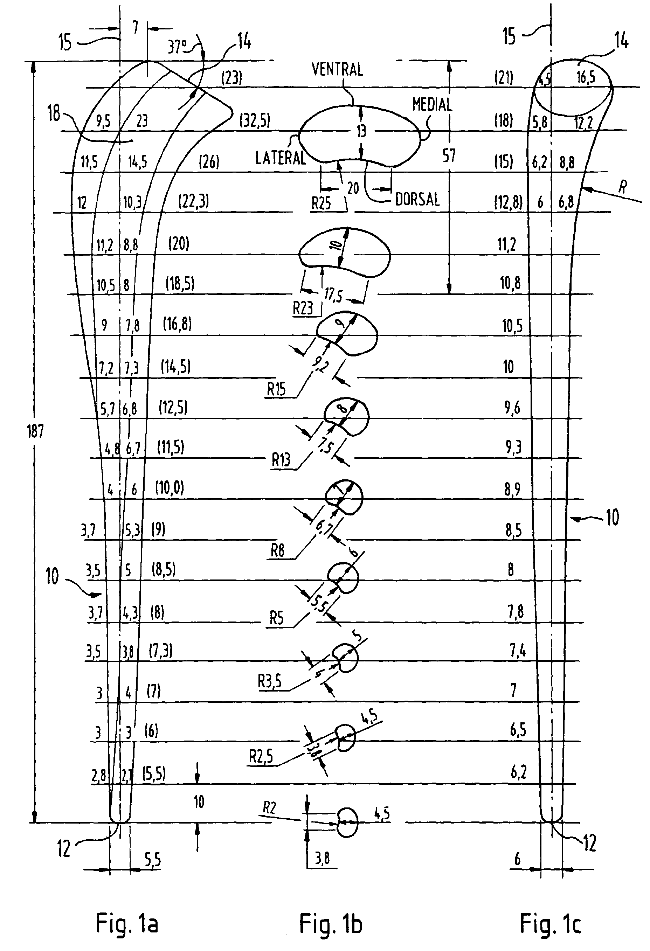

Die Figuren zeigen Ausführungsformen des erfindungsgemäßen Femurstiels etwa im Maßstab 1:1. Sämtliche Maße in Figur 1 sind in mm, falls nicht anders angegeben.

Figur 1A zeigt den Femurstiel 10 mit einer Prothesenspitze 12 und einem Halsansatz 14 sowie einer Konstruktionsachse 15. Außerdem ist in Figur 1A eine konkave Ausnehmung 18 sichtbar, die im proximalen Bereich auf der dorsalen Fläche angeordnet ist und sich nach distal kontinuierlich wendelförmig nach lateral hin verdreht bzw. verwringt.

Die auf der rechten Seite von Figur 1A in Klammern angegebenen Zahlen bedeuten die Gesamtbreite des Femurstiels im Schnitt in der jeweiligen Axialposition, die anderen Zahlen der jeweiligen Reihe bedeuten den Anteil lateral bzw. medial von der Achse 15. Diese Daten sind gute Anhaltspunkte für die Massenverteilung der Prothese lateral bzw. medial von der Achse.

Aus Figur 1A ist weiter entnehmbar, daß die Gesamtlänge des Femurstiels etwa 187 mm, der Durchmesser im distalen Bereich etwa 5,5 mm, der seitliche Abstand zwischen der Achse 15 und dem proximal höchsten Punkt des Stiels etwa 7 mm und der Winkel zwischen der Achse 15 und der Schenkelhalsachse, d.h. zwischen der Achse 15 und der proximalen Endfläche des Femurstiels, etwa 37° beträgt.

Figur 1B zeigt die Schaftquerschnitte in verschiedenen Axialpositionen. Dabei ist deutlich erkennbar, daß der Querschnitt im proximalen Bereich im wesentlichen quer-oval bzw. elliptisch mit langer Achse in medial-lateraler Richtung ist, und eine konkave Ausnehmung auf der dorsalen Fläche aufweist. Zum distalen Ende hin wird der Querschnitt kleiner und "dreht" sich kontinuierlich, so daß am distalen Ende die lange Achse des elliptischen Querschnitts sich von ventral nach dorsal erstreckt und der Querschnitt des implantierten Schafts sagittal-oval ausgebildet ist. Die konkave Ausnehmung dreht sich dabei ebenfalls von dorsal nach lateral. Die Breite sowie der Radius der konkaven Ausnehmung nehmen dabei ebenfalls kontinuierlich von proximal nach distal ab, wobei die Breite der Ausnehmung im proximalen Bereich etwa 20 mm und im distalen Bereich etwa 3,8 mm beträgt und der Radius im Zentrum der Ausnehmung von etwa 25 mm im proximalen Bereich auf etwa 2 mm im distalen Bereich abnimmt. Der Übergang zwischen der Ausnehmung und der verbleibenden Wand des Prothesenstiels ist jeweils abgerundet, wobei in Figur 1B die Rundungsradien eingezeichnet sind. In Figur 1B ist ferner der kleinste Durchmesser des Prothesenquerschnitts, jeweils etwa vom Zentrum der Ausnehmung aus gemessen, eingezeichnet, der von etwa 13 mm im proximalen Bereich auf etwa 4,5 mm im distalen Bereich abnimmt.

Im Schnittbild gemäß Figur 1C (Ansicht von medial) zeigen die Zahlen auf der linken Seite ebenfalls die gesamte Breite des Querschnitts in der jeweiligen Axialposition, die weiteren Zahlenreihen im proximalen Bereich geben die Verteilung des Querschnitts dorsal (links in Fig. 1C) bzw. ventral von der Achse 15 an. Hieraus ist ersichtlich, daß im proximalen Bereich der Massenschwerpunkt des Schafts ventral der Achse des Schafts liegt und eine Krümmung derart ausgebildet ist, daß der Krümmungsmittelpunkt ventral (vorne) liegt. Dies ist in Figur 1C durch den Krümmungsradius "R" angedeutet. Wie aus Figur 1B entnehmbar, erstreckt sich die Krümmung über etwa 57 mm vom proximalen Ende des Femurstiels.

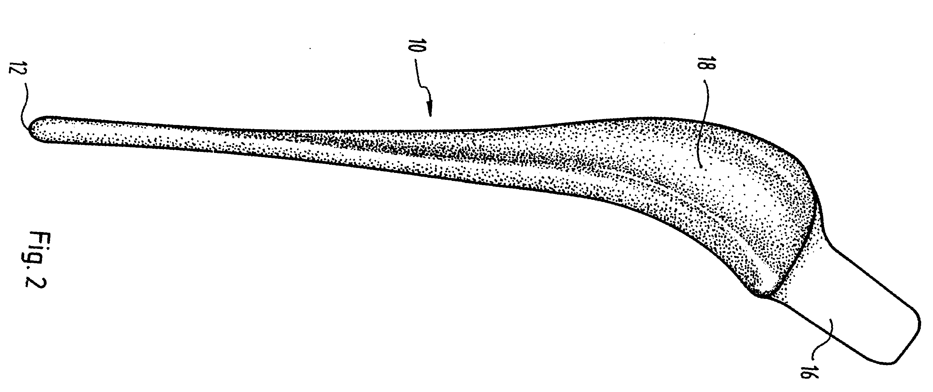

Figur 2 zeigt die Ansicht einer erfindungsgemäßen Prothese 10 von dorsal. Die Prothese weist einen konischen Hals 16 zum Aufsatz verschiedener Kugelköpfe auf. In Figur 2 ist die konische Ausnehmung oder Rinne 18 deutlich erkennbar, die im proximalen Bereich auf der dorsalen Fläche verläuft und sich nach distal hin kontinuierlich zur lateralen Seite hin dreht oder "verwringt". Ähnlich wie aus den Querschnitten gemäß Figur 1B ersichtlich, dreht sich die Rinne 18 von proximal nach distal insgesamt über einen Winkel von etwa 90°.

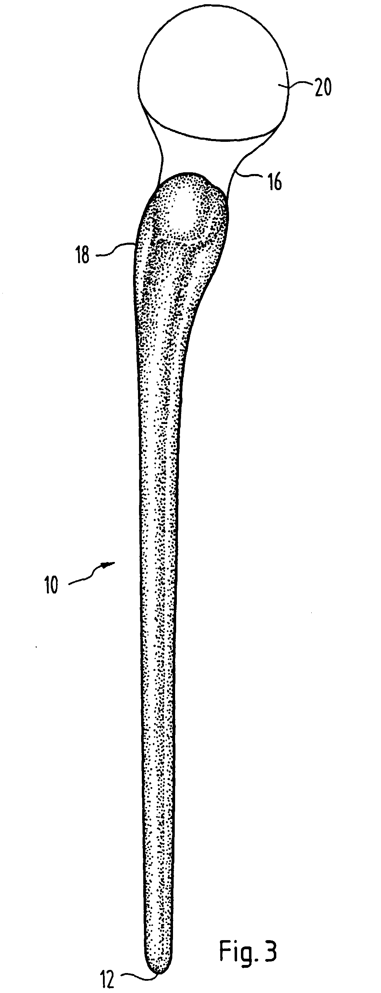

Figur 3 zeigt einen Femurstiel 10 mit Hals 16 und festem Kopf 20 (Standardprothese) in der Ansicht von medial (Linksprothese). Der Femurstiel von Figur 3 entspricht dem Femurstiel gemäß Figur 2. Die Rinne 18 ist im proximalen Bereich auf der dorsalen Seite des Femurstiels erkennbar und dreht sich in Distalrichtung nach lateral, d.h. in die Zeichenebene hinein.

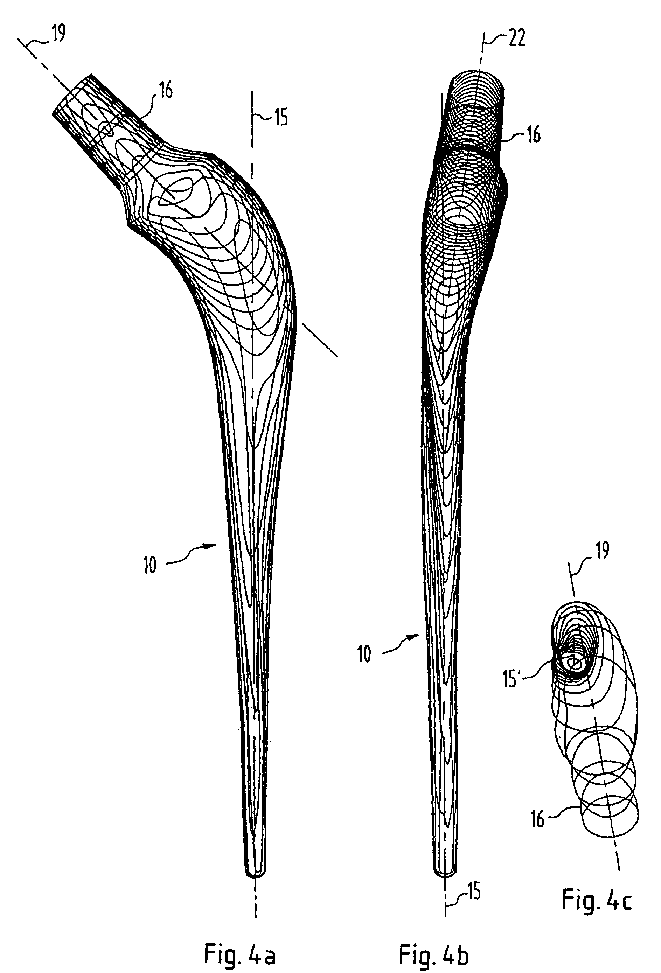

Figur 4A zeigt einen erfindungsgemäßen Prothesenstiel 10 mit Hals 16 (Linksprothese) im Strahlengang anterio-posterior in CAD-Darstellung. Der Hals 16 ist in Richtung der Schenkelhalsachse 19 ausgerichtet. Bei der dargestellten Prothese beträgt der Winkel zwischen der Achse 15 (Einschlagachse der Prothese) und der Schenkelhalsachse 19, die sich senkrecht zur proximalen Querschnittsfläche des Stiels 10 erstreckt, etwa 45°.

In der Darstellung der Prothese im axialen Strahlengang gemäß Figur 4B ist ferner entnehmbar, daß der Winkel zwischen der Achse 15 und der Schenkelhalsachse 22 in dieser Darstellung aufgrund der Krümmung des Prothesenstiels im proximalen Bereich nach ventral etwa 9° beträgt.

In der schematischen Draufsicht der Prothese in Schnittdarstellung gemäß Figur 4C sind mehrere Prothesenquerschnitte eingezeichnet, aus denen ebenfalls die Verwringung des Stielquerschnitts von proximal nach distal erkennbar ist. In Figur 4C ist ebenfalls die Schenkelhalsachse 19 eingezeichnet, sowie die Einschlagachse 15' angedeutet.

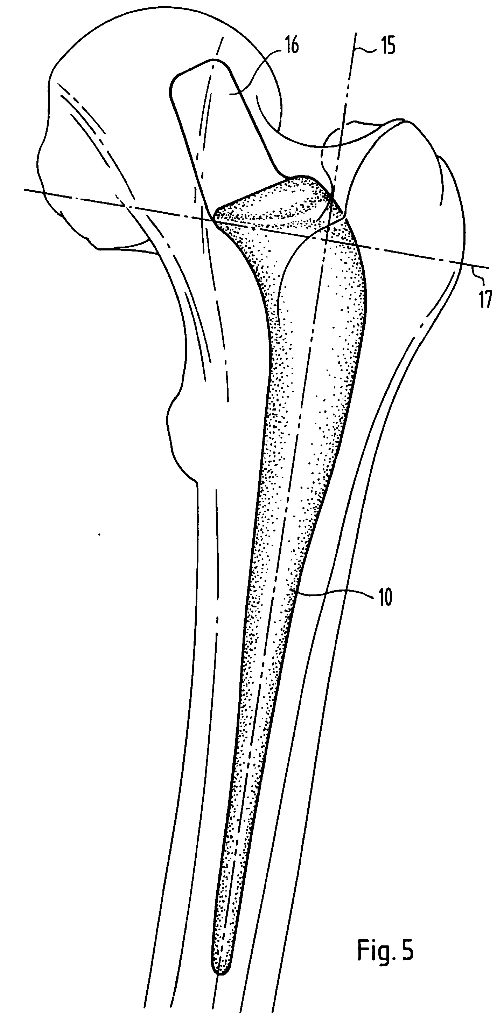

Figur 5 zeigt einen erfindungsgemäßen Femurstiel 10 mit konischem Hals 16 im Strahlengang anterio-posterior (Schnittansicht von vorne, Linksprothese) in implantiertem Zustand. Die Achse 15 entspricht der Medianachse des Femur bzw. der Implantationsachse. Außerdem ist in Figur 5 eine Linie 17 erkennbar, entlang der gegebenenfalls der Kopfteil verschiebbar ist, wie z.B. in der PCT/EP 92/01925 beschrieben.

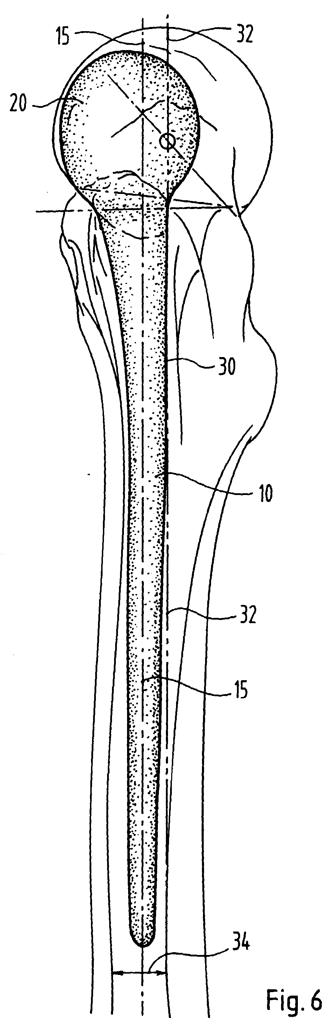

Figur 6 zeigt in implantiertem Zustand einen erfindungsgemäßen Femurstiel 10 mit Kopf 20 in der Ansicht von lateral (axialer Strahlengang). In Figur 6 ist neben der Achse 15, die der Konstruktionsachse der Prothese bzw. der Implantationsachse und der Medianachse des Femur entspricht, auch eine Tangente 32 eingezeichnet, die im axialen Strahlengang an die dorsale Wand des Schenkelhalses und die dorsale Wand des Femurmarkkanales angelegt wird. Die Achse 15 und die Tangente 32 verlaufen im wesentlichen parallel, die Abweichung sollte nicht mehr als ± 5 %, vorzugsweise nicht mehr als ± 3 % betragen. Mit 34 ist in Fig. 6 der Isthmus, d.h. die engste Stelle des Markkanals, gekennzeichnet.

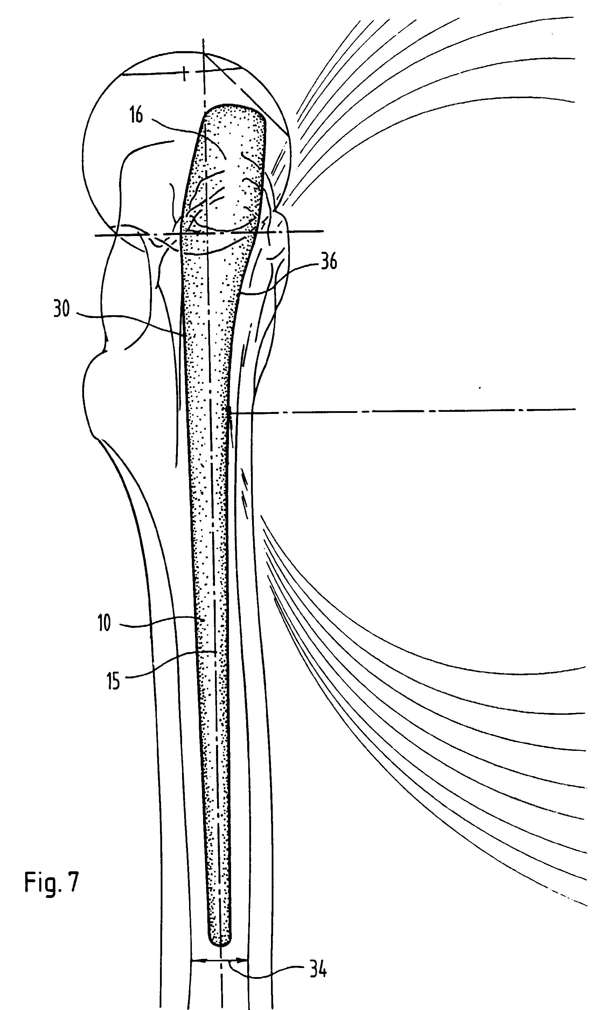

Figur 7 zeigt in ähnlicher Weise wie Figur 6 einen erfindungsgemäßen Femurstiel 10 mit konischem Hals 16 im axialen Strahlengang (Ansicht von medial). Aus Figur 7 ist insbesondere der gerade Verlauf der Dorsalfläche 30 des Stiels im proximalen Bereich und die Krümmung der Ventralfläche 36 im proximalen Bereich entnehmbar. An die Ventralfläche sind beispielhaft mehrere Krümmungsradien angelegt, die vorzugsweise zwischen 80 und 150 mm betragen. Der Krümmungsmittelpunkt liegt dabei ventral. Die Krümmung im proximalen Bereich verläuft parabelförmig, und der Krümmungsradius verkleinert sich im wesentlichen kontinuierlich von distal nach proximal.

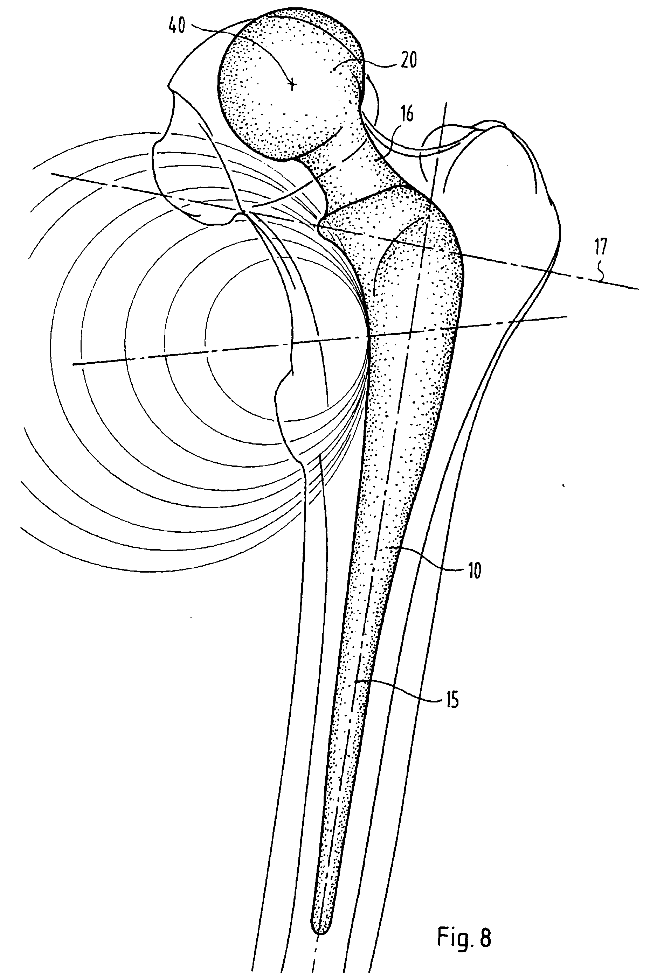

Figur 8 zeigt in ähnlicher Ansicht wie Figur 5 einen erfindungsgemäßen Femurstiel 10 mit Hals 16 und festem Kopf 20 (Standardprothese). Mit 40 ist das Rotationszentrum bezeichnet. Aus Figur 8 ist ersichtlich, daß die Prothese zentral im Femurmarkkanal verankert ist. Aus Figur 8 ist auch die Krümmung des Schafts im proximalen Bereich auf der medialen Seite erkennbar, wobei in Figur 8 beispielhaft mehrere Krümmungsradien eingetragen sind, die vorzugsweise zwischen 25 und 55 mm, besonders bevorzugt zwischen 36 und 40 mm betragen. Die Krümmung verläuft dabei parabelförmig, und der Krümmungsradius verkleinert sich im proximalen Bereich im wesentlichen von distal nach proximal.

## Patentansprüche

1. Femurstiel (10) einer Hüftgelenksendoprothese, im Frontalschnitt (Fig. 8) so gekrümmt, daß der Krümmungsmittelpunkt im proximalen Bereich des Stieles medial liegt, durch folgende Merkmale gekennzeichnet:
- der Femurstiel (10) ist im proximalen Bereich im Querschnitt an seiner Ventralseite konvex und an seiner Dorsalseite konkav oder abgeflacht,
- der Femurstiel weist vorzugsweise über seine gesamte Länge, in jedem Fall in seinem proximalen Bereich eine konkave Ausnehmung oder Rinne (18) oder eine Abflachung auf, die sich nach distal verwringt in der Art, daß sich die im proximalen Bereich dorsale Fläche in Richtung auf die Prothesenspitze der lateralen Wand des Markkanales zuwendet,
- die dorsale Fläche des Stieles (10) ist im Sagittalschnitt gerade,
- die ventrale Fläche (36) des Stieles (10) ist im Sagittalschnitt im proximalen Drittel des Stieles so gekrümmt, daß der Krümmungsmittelpunkt ventral liegt.

2. Femurstiel (10) nach Anspruch 1, wobei die Verwindung der dorsalen Fläche (30) bis 195°, vorzugsweise 45 bis 135°, besonders bevorzugt 80 bis 100°, und ganz besonders bevorzugt 90° (Fig. 1b) beträgt, und vorzugsweise bei einem linken Femurstiel kontinuierlich nach distal im Uhrzeigersinn, bei einem rechten Femurstiel im Gegenuhrzeigersinn verläuft.

3. Femurstiel (10) einer Hüftgelenksendoprothese nach den Ansprüchen 1 und 2, dessen konkave Ausnehmung, Rinne oder Abflachung in axialer Richtung verläuft.

4. Femurstiel (10) nach Anspruch 3, wobei die Drehung 10 bis 170°, vorzugsweise 45 bis 135°, besonders bevorzugt 80 bis 100°, und ganz besonders bevorzugt etwa 90° (Fig. 1b) beträgt.

5. Femurstiel (10) einer Hüftgelenksendoprothese nach Anspruch 1, welcher eine gerade Stielachse (15) aufweist, die parallel zu einer Tangente (32) verläuft, die im axialen Strahlengang eines Röntgenbildes an die dorsale Wand des Schenkelhalses (35) und die dorsale Wand des Femurmarkkanales anliegt.

6. Femurstiel (10) einer Hüftgelenksendoprothese nach Anspruch 1, deren Konstruktionsachse und/oder deren Ventralfläche (36) im axialen Strahlengang des Röntgenbildes des Stieles im proximalen Drittel des Stieles so gekrümmt ist, daß der Krümmungsmittelpunkt ventral liegt.

7. Femurstiel (10) nach Anspruch 6, wobei die Krümmung einen Radius zwischen 60 und 180 mm, vorzugsweise zwischen 80 und 150 mm, besonders bevorzugt zwischen 120 und 130 mm aufweist.

8. Femurstiel (10) nach Anspruch 6, wobei sich der Krümmungsradius von distal nach proximal vorzugsweise kontinuierlich verkleinert.

9. Femurstiel (10) nach Anspruch 1, wobei im seitlichen, axialen Strahlengang des Röntgenbildes (Fig. 6) die Dorsalfläche des Stieles im proximalen Bereich, vorzugsweise im proximalen Drittel, einer geraden Fläche anliegt.

10. Femurstiel (10) einer Hüftgelenksendoprothese nach Anspruch 1, welcher im anterioposterioren Strahlengang im Röntgenbild so gekrümmt ist, daß der Krümmungsmittelpunkt im proximalen Bereich des Stieles medial (Fig. 8) liegt.

11. Femurstiel (10) nach Anspruch 10, wobei der Radius der Krümmung 15 bis 85 mm, vorzugsweise 25 bis 55 mm, besonders bevorzugt 36 bis 40 mm beträgt.

12. Femurstiel (10) nach Anspruch 10 oder 11, wobei die Krümmung parabelförmig verläuft und der Krümmungsradius sich im wesentlichen kontinuierlich von distal nach proximal verkleinert.

13. Femurstiel (10) einer Hüftgelenksendoprothese nach den Ansprüchen 1 bis 5, die medial als breit aufliegende und nach medial konvexe U-Form ausgebildet ist und insgesamt einen horizontal quer-ovalen bis rechteckigen Querschnitt mit deutlich abgerundeten Kanten aufweist.

14. Femurkomponente einer Hüftgelenksendoprothese mit einem Femurstiel nach einem der Ansprüche 1 bis 13, einem Prothesenhais, einem Konus für die Kopfaufnahme und/oder einem Prothesenkopf.

## Claims

1. A femoral shaft (10) of a hip joint prosthesis which in a front sectional view (Fig. 8) is curved so that the center of curvature is medial in the proximal area of said shaft, characterized in that:
said femoral shaft (10) being convex at its ventral side in its proximal area in sectional view while being concave (18) or flattened at its dorsal side;
wherein said femoral shaft including preferably along its entire length and at least in its proximal area a concave recess or groove (19) or a flattened portion, which is distorted distally such that the dorsal area in said proximal area being oriented in the direction of the front end of said prosthesis of the lateral wall of the marrow channel;
the dorsal area of the shaft (10) being straight in sagittal sectional view;
the ventral area (36) of said shaft (10) in sagittal sectional view being curved in the proximal third of said shaft such that the center of curvature is positioned ventrally.

2. The femoral shaft (10) according to claim 1, wherein said distortion of said dorsal area being between 15 and 195°, preferably between 45 and 135°, more preferably between 80 and 100° and most preferably 90° (Fig. 1b), and wherein preferably being continually distally clockwise for a left femoral shaft and counter-clockwise for a right femoral shaft.

3. The femoral shaft (10) of a hip joint prosthesis according to claims 1 and 2, wherein said axially disposed concave recess, groove or flattened portion being positioned in said proximal area on said dorsal area of said femoral shaft and being rotatable laterally to said front end (12) of said prosthesis.

4. The femoral shaft (10) according to claim 3, wherein said rotation is between 10 and 170°, preferably between 45 and 135°, more preferably between 80 and 100° and most preferably 90° (Fig. 1b).

5. The femoral shaft (10) of a hip joint prosthesis according to claim 1 including a straight shaft axis (15) which in the axial beam path is parallel to a tangent (32) being adjacent to said dorsal wall of the femoral neck (35) and said dorsal wall of said femoral marrow channel.

6. The femoral shaft (10) of a hip joint prosthesis according to claim 1, wherein its structural axis and/or its ventral area (36) in said axial beam path of the X-ray image of said shaft being curved in the proximal third of said shaft, such that the center of curvature is disposed ventrally.

7. The femoral shaft (10) according to claim 6, wherein said curvature includes a radius between 60 and 180 mm, preferably between 80 and 150 mm and most preferably between 120 and 130 mm.

8. The femoral shaft (10) according to claim 6, wherein said radius of curvature continually becoming smaller from the distal end to the proximal end.

9. The femoral shaft (10) according to claim 1, wherein the dorsal area of said shaft in the proximal region, preferably in the proximal third, in the sidewise axial beam path of said X-ray image being adjacent to a straight area.

10. The femoral shaft (10) of a hip joint prosthesis according to claim 1, wherein said shaft being curved in the anterior-posterior beam path in the X-ray image such that the center of curvature is disposed medially in said proximal region of said shaft (Fig. 8).

11. The femoral shaft (10) according to claim 10, wherein said radius of curvature is between 15 and 85 mm, preferably between 25 and 55 m and most preferably between 36 and 40 mm.

12. The femoral shaft (10) according to claims 10 or 11, wherein said curvature being parabolic, and wherein said radius of curvature generally continuously becoming smaller from the distal end to the proximal end.

13. The femoral shaft (10) of a hip joint prosthesis according to claims 1 to 5, medially being formed as a flat supported and medially convex U-shape, and wherein in all it includes a horizontally lateral-oval to square profile with clearly rounded edges.

14. A femoral component of a hip joint prosthesis including a femoral shaft according to one of the claims 1 to 13, a prosthesis neck, a cone for receiving the head and/or a prosthesis head.

## Revendications

1. Tige fémorale (10) d'une endoprothèse de l'articulation de la hanche vue en coupe frontale (fig. 8), recourbée de telle sorte que le point central de la courbure se situe médialement dans la région proximale de la tige, caractérisée par les éléments suivants:
- la tige fémorale (10) dans sa région proximale et vue en coupe transversale est convexe à sa partie ventrale et concave à sa partie dorsale (18) ou bien aplatie,
- la tige fémorale présentant électivement sur toute sa longueur et dans tous les cas au niveau de sa région proximale une cavité ou une gouttière concave (19) ou bien encore un aplatissement qui réalise une torsion vers la partie distale de telle sorte que la surface dorsale de la région proximale se dirige vers la paroi latérale du canal médullaire, en direction de la pointe de la prothèse,
- la surface dorsale de la tige (10) est plane vue en coupe sagittale,
- la surface ventrale (36) de la tige (10) vue en coupe sagittale est recourbée au niveau du tiers proximal de la tige de telle sorte que le point central de la courbure se situe ventralement.

2. Tige fémorale (10) selon revendication 1, la torsion de la surface dorsale étant de 15 à 195°, de préférence de 45 à 135°, et de manière particulièrement préférentielle de 80 à 100°, et tout particulièrement préférentielle de 90° (fig. 1b), et allant de préférence dans le cas d'une tige fémorale gauche de manière continue vers la partie distale dans le sens des aiguilles d'une montre, dans le cas d'une tige fémorale droite dans le sens contraire aux aiguilles d'une montre.

3. Tige fémorale (10) d'une endoprothèse de l'articulation de la hanche selon les revendications 1 et 2, la cavité concave, la gouttière ou l'aplatissement orientés de manière axiale étant disposés dans la région proximale contre la surface dorsale de la tige fémorale, tournent latéralement en direction de la pointe de la prothèse (12).

4. Tige fémorale (10) selon revendication 3, la torsion de la surface dorsale étant de 10 à 170°, de préférence de 45 à 135°, et de manière particulièrement préférentielle de 80 à 100°, et tout particulièrement préférentielle d'environ 90° (fig. 1b).

5. Tige fémorale (10) d'une endoprothèse de l'articulation de la hanche selon revendication 1, présentant un axe de la tige (15) qui s'étend parallèlement à une tangente (32) qui selon le trajet des rayons d'une radiographie est appliqué contre la paroi dorsale du col du fémur (35) et contre la paroi dorsale du canal médullaire du fémur.

6. Tige fémorale (10) d'une endoprothèse de l'articulation de la hanche selon revendication 1, dont l'axe de construction et/ou dont la surface ventrale (36) est recourbé(e) de telle manière selon le trajet axial des rayons de la radiographie de la tige au niveau du tiers proximal de la tige que le point central de la courbure se situe ventralement.

7. Tige fémorale (10) selon revendication 6, la courbure présentant un rayon de 60 à 180 mm, de préférence de 80 à 150 mm, de manière particulièrement préférentielle de 120 à 130 mm.

8. Tige fémorale (10) selon revendication 6, le rayon de la courbure se réduisant de préférence continuellement depuis la partie distale et en direction de la partie proximale.

9. Tige fémorale (10) selon revendication 1, dans le trajet latéral et axial des rayons de la radiographie (fig. 6) la surface dorsale de la tige dans la région proximale étant appliquée de préférence dans le tiers proximal contre une surface plane.

10. Tige fémorale (10) d'une endoprothèse de l'articulation de la hanche selon revendication 1, recourbée de telle manière dans le trajet antéro-postérieur de la radiographie que le point central de la courbure se situe médialement (fig. 8) dans la région proximale de la tige.

11. Tige fémorale (10) selon revendication 10, le rayon de la courbure étant compris entre 15 à 85 mm, de préférence entre 25 à 55 mm, et de manière particulièrement préférentielle entre 36 à 40 mm.

12. Tige fémorale (10) selon revendication 10 ou 11, la courbure étant de forme parabolique et le rayon de la courbure diminuant sensiblement et continuellement depuis la partie distale et vers la partie proximale.

13. Tige fémorale (10) d'une endoprothèse de l'articulation de la hanche selon revendications 1 à 5, étant médialement réalisée comme se trouvant appliquée sur une surface large et prenant une forme en U convexe médialement et se présentant dans l'ensemble selon une coupe horizontale transverse-ovale à rectangulaire avec des bords nettement arrondis.

14. Composante fémorale d'une endoprothèse de l'articulation de la hanche avec une tige fémorale selon revendications 1 à 13, un col de prothèse, un cône pour recevoir la tête et/ou une tête prothétique.
